(19) Europäisches Patentamt

European Patent Office

Office européen des brevets

(11) **EP 0 840 127 B1**

(12) **EUROPEAN PATENT SPECIFICATION**

(45) Date of publication and mention
of the grant of the patent:
**22.12.2004   Bulletin 2004/52**

(51) Int Cl.⁷: **G01N 33/82**

(21) Application number: **97308698.6**

(22) Date of filing: **30.10.1997**

(54) **Table and method for assessing vitamin mineral profile**

Tabelle und Verfahren zur Beurteilung von Vitamin-Mineral Profildaten

Table et procédé pour l'évaluation de profil vitamines-minéraux

(84) Designated Contracting States:
**CH DE DK FR GB LI NL**

(30) Priority: **30.10.1996  JP 1106696**
**07.02.1997  JP 2530897**

(43) Date of publication of application:
**06.05.1998  Bulletin 1998/19**

(73) Proprietor: **Nikken Foods Company Limited**
**Fukuroi-shi, Shizuoka (JP)**

(72) Inventor: **Ochi, Hirotomo**
**Fukuroi-shi, Shizuoka (JP)**

(74) Representative: **Ablewhite, Alan James**
**MARKS & CLERK,**
**57/60 Lincoln's Inn Fields**
**London WC2A 3LS (GB)**

(56) References cited:
**DE-A- 3 511 832**     **DE-A- 3 819 248**
**GB-A- 2 177 839**     **GB-A- 2 288 257**
**JP-A- 57 190 265**     **RU-C- 2 064 778**

- **VANDERVEEN, V, ET AL: "Remington's**
**Pharmaceutical Sciences " 1985 , MACK , PA,**
**USA XP002054039 * page 1002 - page 1005; table**
**1 ***

## EP 0 840 127 B1

**Description**

BACKGROUND OF THE INVENTION

Field of the Invention

The present invention relates to the development of a method and a table for assessing vitamin mineral profile. The invention will be used for the purpose of getting information about the vitamin mineral profile in a test subject in order to determine whether vitamin and mineral are excessive or insufficient. By measuring the indicator substances that include a variety of vitamins and minerals in blood, which suggest the human health state and the extent of aging process, the measured values are represented in numerical figures and recorded in the form of a table for assessment. The resulting numerical figures are then compared with normal numerical figures attained from healthy subjects.

Description of the Related Art

Vanderven V. et al., in 'Remington's Pharmaceutical Sciences' (1985) 1002-1005, disclose a Table of recommended daily dietary allowances for fat- and water-soluble vitamins and minerals (p. 1004, Table I) and a Table presenting the estimated safe and adequate daily dietary intakes of diverse additional vitamins and minerals (p. 1004, Table II).

JP 57 190 265 discloses a method of examining the nutritional balance of ingested nutrients in foods being eaten comprising a check list of foods in which a nutrient list of various foods in which average amount of each food eaten and the amounts of various nutrients such as vitamins, calcium, contained in each nutrient in food eaten are given.

DE 38 19 248 discloses an electronic apparatus to input data about intake of various nutrients including vitamins and minerals.

Conventionally, specific vitamins (for example, vitamins A, B1, B2, C, etc.) and minerals (for example, iron) have been measured at health check, but the excess or shortage thereof has not been assessed frequently. Even if such assessment has been made, various types of advice have been suggested on food intake alone.

Also at laboratory tests, the indicator substances of a variety of vitamins and minerals have been measured in blood for detection or treatment of a disease, to make diagnosis and treatment on the basis of the excess or deficiency of the substances compared with the standard values of the substances.

Such conventional method has some effect in diagnosing and treating the disease in a symptomatic fashion, by examining the health state or the conditions of the disease, but such method cannot assess overall health state or the extent of aging. The method can neither give any suggestion for the control and amelioration of the health state or for the control of aging, disadvantageously. Though the symptomatic treatment can measure the indicator substances appropriately for the treatment of the diseased conditions and can practically treat the conditions, however this approach can not be used in the control of the aging process or for the prevention of adult diseases prior to their onset. Specifically, as of now an indicator table or graph by which overall assessment can be made has not been proposed yet.

SUMMARY OF THE INVENTION

In accordance with the present invention, the problems stated in the before described Description of the Related Art can be overcome, by measuring and numerically representing the amounts of a variety of indicator substances of vitamin and mineral in the blood sample of the test subject, and recording the numerical figures in an assessment table or graphically representing the figures. The values are thereafter compared with the numerical figures in normal subjects to readily grasp information about the vitamin mineral profile as to whether a variety of indicator substances of vitamin and mineral are excessive or insufficient. Additionally, the assessment of aging control in humans can be carried out readily on the basis of the resulting table or graph.

More specifically, the present invention is a table for assessing the vitamin mineral profile, which is designed by representing sequentially indicator substance items of vitamin and mineral in blood in abscissa, which items suggest the status of human health, representing the numerical figures indicating the degree of excess or deficiency of indicator substances of vitamin and mineral in ordinates, defining the numerical figures of the standard values of vitamin and mineral on the axis of ordinates, and arranging the standard range of each indicator substance above and below the center where the numerical figure corresponding to the standard value is placed.

The status of human health includes the status of aging progress and the status of the progress of adult diseases, in addition to general and overall status of human health.

The standard range can be defined by adjusting the numerical figure of the normal value of each indicator substance to the numerical figure of the standard value and setting the lower limit of the standard value, corresponding to the lower limit of the normal range of each indicator substance, and the upper limit of the standard value, corresponding to the upper limit of the normal range, while these limits should interpose the numerical figure of the standard value.

The present invention proposes another table for assessing vitamin mineral profile, which is produced by representing sequentially indicator substance items of water-soluble vitamin, fat-soluble vitamin and mineral in blood in abscissa, which items suggest the status of human health, representing the numerical figures indicating the degree of excess or shortage of indicator substances of vitamin and mineral in ordinates, defining the numerical figures of the standard values of vitamin and mineral on the axis of ordinates, adjusting a standard value corresponding to the normal value of each indicator substance to the numerical figures of the standard value, arranging the standard range of each indicator substance defined between the lower limit standard value corresponding to the lower limit of the normal range of each indicator substance and the upper limit standard value corresponding to the upper limit of the normal range thereof above and below the center where the numerical figure corresponding to the standard value is placed, and individually setting the safety range and abnormal range of water-soluble vitamin and abnormal ranges of fat-soluble vitamin and mineral.

The normal value described above is a mean value within the normal range of each indicator substance of vitamin or mineral, as published by a laboratory test center. The standard range corresponds to the normal range of each indicator substance of vitamin or mineral, as published by the laboratory test center, and represented on the table for assessment wherein the standard value is placed at the center and the range is represented in a range above and below the standard value.

Because each indicator substance has an intrinsic normal range, some indicator substance has a relatively wide standard range above and below the center where the standard value is placed on the table for assessment, while some indicator substance has a relatively narrow standard range above and below the center.

As has been described above, the standard range corresponds to the normal range of each indicator substance of vitamin or mineral, published by the laboratory test center, and therefore, the standard range can be approved as the numerical standard range thereof in normal subjects. Alternatively, the abnormal range described above is a range under possible influence of some disease, because the range is significantly departed from the standard range. Additionally, the safety range means a range also significantly departed from the numerical standard range but possibly with no pathological problem. As described above, the safety range is defined only for water-soluble vitamin. Even if water-soluble vitamin is excessive, the vitamin is excreted from human bodies, and therefore, any outcome suggesting that the level of water-soluble vitamin in a test subject is above the standard range is not problematic, pathologically. Therefore, the safety range above the standard range can be defined for only water-soluble vitamin.

The indicator substances in blood, suggesting the human health status, include what will be described below. More specifically, the vitamin includes vitamin A, vitamin B1, vitamin B2, vitamin B6, vitamin B12, folic acid, vitamin C, vitamin E, carotene, nicotinic acid, biotin and inositol. Among them, vitamin C, vitamin B1, vitamin B2, folic acid, vitamin B6, vitamin B12, nicotinic acid, biotin and inositol are water-soluble vitamins, while the remaining are fat-soluble vitamins. Furthermore, mineral includes sodium, potassium, calcium, magnesium, phosphorus, nickel, silicon, aluminium, iron, zinc, copper, manganese, boron, molybdenum, cobalt, chromium, iodine, fluorine, selenium and vanadium.

It has been known conventionally that anti-oxidant vitamins and micronutrient minerals have close relation to human health status, aging, and adult diseases. However, the progress of aging and adult diseases cannot be assessed in a simple manner on the basis of one vitamin or mineral, but the progress thereof should be assessed on the basis of a plurality of more vitamins and minerals. In accordance with the present invention, therefore, a great number of vitamins and minerals as described above are defined as indicator substances, whereby the stage of the progress of aging and adult diseases and the health status can entirely be assessed.

A method for assessing the vitamin mineral profile in a test subject by using the table for assessing the vitamin mineral profile in accordance with the present invention will be described as follows.

More specifically, getting the data of indicator substances of vitamin and mineral suggesting the health status of a human test subject in blood on the basis of the blood test outcome of the subject, numerically representing the data, recording the numerical figures on the table for assessing the vitamin mineral profile in accordance with the present invention, the degree of excess or shortage of each indicator substance is numerically assessed or the vitamin mineral profile in the test subject is visually assessed.

The data recovered on the basis of the blood test outcome in the test subject means a measured value of each indicator substance, as assessed by routine blood test. When representing the data, namely the measured value, on the basis of the numerical figure to be recorded, on the table for assessing vitamin mineral profile in accordance with the present invention, the following formula should be used for conversion.

1. Provided that the measured value at the blood test of the test subject is above the upper limit of the normal range of the indicator substance;

$$\text{(measured value - the upper limit of normal range)} \div \text{the upper}$$

$$\text{limit of normal range} \times 100 + 100$$

2. Provided that the measured value at the blood test of the test subject is below the lower limit of the normal range of the indicator substance;

$$100 - [(\text{the lower limit of normal range - measured value}) \div \text{the}$$

$$\text{lower limit of normal range} \times 100]$$

The above conversion formula is a conversion formula, with the provisions that the numerical figure corresponding to the standard value, namely normal value of each indicator substance, which is represented on the axis of ordinates in the table for assessing vitamin mineral profile, is defined as 100. More specifically, provided that the numerical figure corresponding to the standard value, namely normal value of each indicator substance, which is represented on the axis of ordinates therein, is defined as 50, the conversion formula (1) is corrected as follows; (measured value - the upper limit of normal range) ÷ the upper limit of normal range × 100 + 50. The conversion formula (2) is corrected as follows; 50 - [(the lower limit of normal range - measured value) ÷ the lower limit of normal range × 100].

Because the ratio of the excess or shortage of the measured value, relative to the upper limit or lower limit of normal value, is represented as percentage, it is more handy that the numerical figure corresponding to the standard value, namely the normal value represented on the axis of ordinates, is defined as 100.

The reason why the measured value should be converted as described above will be described below. Specifically, it is not preferable that the content, namely measured value, of fat-soluble vitamin and mineral in blood among such indicator substances for assessing the state of human health and the progress of aging and adult diseases, is far above or below the normal range. Preferably, the content is within the normal range; as the measured value deviates more from the normal range, adversely, the progress or pathological degree of aging is diagnosed as in progress. Alternatively, even excess water-soluble vitamin is not at all problematic, because the vitamin is routinely excreted from the human body as described above. However, the content of water-soluble vitamin far less below the normal range, is not preferable from the respect of the progress of aging and adult diseases.

So as to readily take a glance as to how much extent measured values from a test subject deviates from the normal range, as has been described above, the measured values are divided as a group above the upper limit of normal range and a group below the lower limit of the normal range to introduce individually numerical figures.

In the table for assessing the status of vitamin and mineral in accordance with the present invention, the indicator substance items of vitamin and mineral in a blood, suggesting the status of human health and the progress of aging, are sequentially depicted in abscissa, while in ordinates, numerical figures representing the excess or deficiency of the individual indicator substance items of vitamin and mineral are depicted. Then, numerical figures corresponding to the standard values of the individual vitamin and mineral are defined on the axis of ordinates, and a numerical figure corresponding to the normal value of each indicator substance is adjusted to the numerical figure of the standard value, and a standard range defined between the lower limit standard value corresponding to the lower limit of the normal range of each indicator substance and the upper limit standard value corresponding to the upper limit of the normal range thereof is arranged. By subsequently representing the measured value of each indicator substance from a test subject as a numerical figure according to the previously described conversion formula and recording the figure on the table for assessing vitamin mineral profile, the excess or shortage of each indicator substance can be judged so readily. If the numerical figure of each indicator substance is graphically represented in addition to the simple representation thereof in dot, the excess or deficiency of each indicator substance can be judged more readily.

In the other table for assessing vitamin mineral profile as proposed in accordance with the present invention, furthermore, items corresponding to individual indicator substances including water-soluble vitamin, fat-soluble vitamin and mineral, suggesting the state of human health and the progress of aging, can be represented sequentially on abscissa, while on ordinates, the numerical figures representing the excess or deficiency of vitamin and mineral as the individual indicator substance items are represented. On the ordinates, additionally, numerical figures corresponding to the standard values of vitamin and mineral are definitely shown. Furthermore, adjusting the numerical figure corresponding to the normal value of each indicator substance to the numerical figure corresponding to the standard value, the standard range interposed by the adjusted figure and defined by the lower limit standard value corresponding to the lower limit of the normal value of each indicator substance and the upper limit standard value corresponding to the upper limit of the normal value thereof is arranged above and below the center of the standard range, which center is occupied by the numerical figure corresponding to the standard value. The safety range and abnormal range of water-soluble vitamin and the abnormal ranges of fat-soluble vitamin and mineral are independently defined. By then representing the measured value of each indicator substance from a test subject as a numerical figure according to the conversion formula and recording the figure on the table for assessing vitamin mineral profile, the information about the status of each indicator substance in the test subject, namely at which state among the state in the standard range, the state in the abnormal range or the state in the safety range the test subject is now on, can be caught by glance.

Based on the outcome according to the method for assessing vitamin mineral profile, the overall health state of a test subject can be checked; the effect of a therapeutic method for aging control and prevention of adult diseases can be examined; and the therapeutic method can appropriately be modified, consequently. Additionally, the overall health state and the status of aging control can be quantitatively examined.

The table for assessment in accordance with the present invention can effectively bring about useful information for totally assessing the excess or shortage of vitamin and mineral in a test subject and analyzing the outcome and treating an appropriate therapy, by graphically recording the information from the blood measurement of the test subject. The table is effective particularly in that the excess or deficiency of vitamin and mineral can be numerically represented, whereby the physical influence of the excess or deficiency of vitamin and mineral can be examined appropriately even if the influence is revealed or not revealed, to sequentially check the stage of the progress of disease or the progress of aging and, appropriately countermeasure against the stage of the progress may be considered.

BRIEF DESCRIPTION OF THE DRAWINGS

Fig. 1 is a plane view depicting graphically the information recovered from the measurement on the table for assessing vitamin mineral profile in accordance with the present invention; and

Fig. 2 is the table for assessing vitamin mineral profile, in which the standard range, safety range and abnormal range preliminarily divided are shown.

The present invention will now be described in the following preferable examples with reference to attached drawings.

Example 1

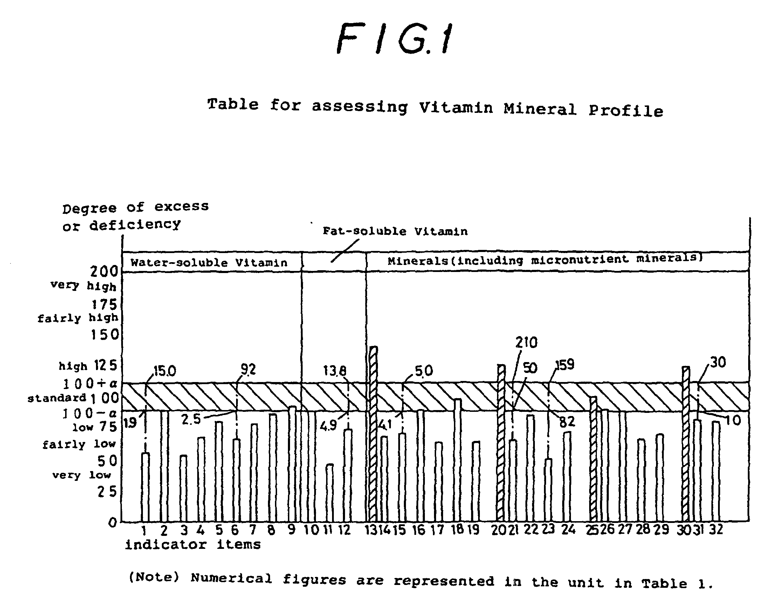

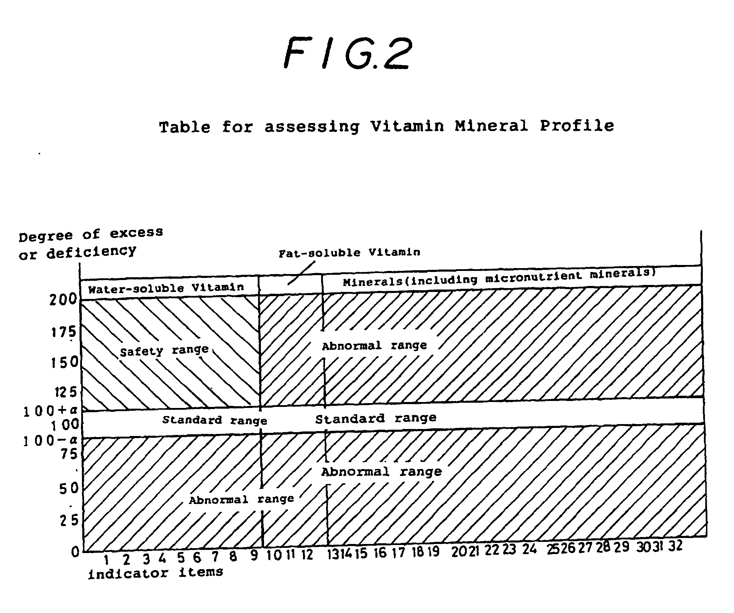

An example of the present invention will be described below with reference to Fig. 1 and Fig. 2. On the figures, the numerical figures representing the excess or deficiency of vitamin and mineral are shown on ordinates; the numbers of the individual indicator substance items of vitamin and mineral are shown on abscissa.

On the ordinates, the numerical figure 100 is defined as the numerical figure corresponding to the standard value, namely normal value (mean value within the normal range of each indicator substance) of each indicator substance of vitamin or mineral. Then, the upper limit and lower limit of the normal range of each indicator substance are numerically represented in the same manner as the manner by which the normal value is defined as 100, correspondingly to the standard value. The numerical figures of individual indicator substances, corresponding to the upper limit and lower limit of their normal ranges, are recorded above and below the numerical figure (100) defined correspondingly to the individual standard values, namely normal values, to arrange the standard range by binding the values together.

In Fig. 1, the standard range is shown in the range of 100 - $\alpha$ to 100 + $\alpha$, because individual indicator substances have different normal ranges, being wide or narrow, and because the substances are represented in different units. Hence, the lower limits and upper limits of the standard ranges of individual indicator substances cannot be defined linearly. For example, vitamin C has a normal range of 1.9 $\mu$g/ml to 15.0 $\mu$g/ml (difference between the upper and lower limits is 13.1 $\mu$g/ml); nicotinic acid has a normal range of 4.7 $\mu$g/ml to 7.9 $\mu$g/ml (difference between the upper and lower limits is 3.2 $\mu$g/ml). Hence, as to the standard ranges thereof above and below the standard value (100), vitamin C has a wider standard range than the standard range of nicotinic acid. In Fig. 1, more specifically, both the upper and lower limits of the standard range are linearly represented for simple representation, but practically, the width from the top to bottom of the standard range varies, depending on the width of the standard range of each indicator substance.

If coloring such standard ranges or individually binding the upper limits of the standard ranges together and the lower limits thereof together with different colors, a readily observable table for assessing vitamin mineral profile can be yielded. Not only numerical figures representing the excess or shortage but also representation of some comment such as "very low" to "very high" on the axis of ordinates can enhance the ready observation of the table, as shown in Fig. 1.

In the table for assessing vitamin mineral profile, vitamins and minerals described in Table 1 below can be used. The numbers represented in the column Graph No. in Table 1 correspond to the numbers of indicator substance items described on the axis of abscissa in the table for assessing the vitamin mineral profile of Fig. 1.

The normal range represented in Table 1 is cited from the numerical figures of healthy subjects at health check, as published by the laboratory test center, which represents the standard range of each indicator substance in numerical figure. As described above, the mean value of the normal range of each indicator substance, namely normal value, should correspond to the standard value (100) described on the axis of ordinates on the table for assessing vitamin mineral profile in Fig. 1, and then, the upper and lower limits of the normal range of each indicator substance are numerically shown in the same manner as for corresponding the normal value to the standard value (100) as described above. Then, the lower limit and upper limit of the standard range, corresponding to the upper and lower limits of the normal range of each indicator substance, are recorded therein to arrange the standard range.

## Table 1

### Indicator substances

| Vitamins | | | Minerals | | |
|---|---|---|---|---|---|
| Graph No. | Title | Normal range (unit) | Graph No. | Title | Normal range (unit) |
| 1 | Vitamin C | 1.9–15.0 $\mu$g/ml | 13 | Sodium | 135–145mEq/l |
| 2 | Vitamin B1 | 18.4–53.1ng/ml | 14 | Potassium | 3.5–50mEq/l |
| 3 | Vitamin B2 | 2.6–3.7ng/ml | 15 | Calcium | 4.1–5.0mEq/l |
| 4 | Vitamin B6 | 3.6–18.0ng/ml | 16 | Magnesium | 1.7–2.6mg/dl |
| 5 | Vitamin B12 | 230–820pg/ml | 17 | Phosphorus | 2.25–2.5mg/dl |
| 6 | Folic acid | 2.5–9.2ng/ml | 18 | Bromine | 270–330$\mu$g/dl |
| 7 | Nicotinic acid | 4.7–7.9$\mu$g/ml | 19 | Silicon | 470–530$\mu$g/dl |
| 8 | Inositol | 2.9–6.7$\mu$g/ml | 20 | Aluminium | 13–17$\mu$g/dl |
| 9 | Biotin | 0.0075–1.73 $\mu$g/dl | 21 | Iron | 50–210$\mu$g/dl |
| 10 | Vitamin A | 30–110$\mu$g/dl | 22 | Zinc | 50–110$\mu$g/dl |
| 11 | Carotene | 50–400$\mu$g/dl | 23 | Copper | 82–159$\mu$g/dl |
| 12 | Vitamin E$\alpha$ | 4.90–13.80$\mu$g/ml | 24 | Manganese | 0.8–2.5$\mu$g/dl |
| | Vitamin E$\beta$ | 0.06–0.2$\mu$g/ml | 25 | Nickel | 2–4$\mu$g/dl |
| | Vitamin E$\gamma$ | 0.1–2.4$\mu$g/ml | 26 | Molybdenum | 0.8–2.5$\mu$g/dl |
| | Vitamin E$\delta$ | less than 0.14 $\mu$g/ml | 27 | Cobalt | 0.007–6$\mu$g/dl |
| | | | 28 | Chromium | less than 1 $\mu$g/dl |
| | | | 29 | Iodine | 2–4$\mu$g/dl |
| | | | 30 | Fluorine | 10–20$\mu$g/dl |
| | | | 31 | Selenium | 10–30$\mu$g/dl |
| | | | 32 | Vanadium | 0.5–23$\mu$g/dl |

When the vitamin mineral profile of a test subject is to be assessed by using the table for assessing vitamin mineral profile, as described in the present Example, the numerical figures recovered from the blood measurement of the test subject are corrected by using the conversion formula and recorded on the table for assessment. In Fig. 1, such numerical figures are shown not only in dot but also in bar graph, whereby the vitamin mineral profile can be checked more readily than the case of simple representation of such numerical figures in dots.

Example 2

Fig. 2 represents the other table for assessing vitamin mineral profile in accordance with the present invention. As in the Example of Fig. 1, the table for assessing vitamin mineral profile of Fig. 2, represents sequentially indicator substance items of water-soluble vitamin, fat-soluble vitamin and mineral in blood on the axis of abscissa, which items suggest the status of human health, and represents the numerical figures indicating the degree of excess or shortage of indicator substances of vitamin and mineral on the axis of ordinates. Additionally, the numerical figures of the standard values of vitamin and mineral are defined as 100 on the axis of ordinates. Then, the standard range of each indicator substance is arranged above and below the center where the numerical figure corresponding to the standard value is placed. As described in Example 1, the line binding the upper standard values or the line binding the lower standard values is not linear, but for simple description, such line is represented linearly in Fig. 2. In the present Example, additionally, the safety range and abnormal range of water-soluble vitamin and the abnormal ranges of fat-soluble vitamin and mineral are arranged individually.

The normal value, safety range and abnormal range are as described above. The reason why the safety range is defined only for water-soluble vitamin is as described above.

When the vitamin mineral profile of a test subject is assessed by using the table for assessing vitamin mineral profile in accordance with the present invention, the numerical figures from the blood measurement of the test subject are preliminarily converted into those by numerical representation according to the conversion formula to be recorded on the table for assessment. Otherwise, the table for assessing vitamin mineral profile of the present Example is printed out on a transparent or semi-transparent sheet, and then, the transparent sheet is overlaid on the table for assessing vitamin mineral profile, as represented in Fig. 1 and recorded with the numerical figures of converted measured values of the test subject.

In such manner, the information as to where the numerical figure of each indicator substance of a test subject is present among the standard range, safety range and abnormal range, can be immediately recovered.

Example of use

A method for assessing the vitamin mineral profile of a test subject by using the table for assessing vitamin mineral profile in accordance with the present invention will be described below.

As described in the Examples 1 and 2, the table for assessing vitamin mineral profile is prepared to record the numerical figures recovered from a test subject on the table. As described in Example 2, the standard range, safety range and abnormal range are assessed by using the table for assessing vitamin mineral profile.

The excess or deficiency of vitamin and mineral has a close relation with the state of human health and adult diseases and additionally a very close relation with aging. Therefore, the possibility of the onset of adult diseases and the extent of aging process are ranked as five stages as described below on the basis of the table for assessing vitamin mineral profile, whereby the prevention of adult diseases and the control of aging can be measured, depending on the individual stages thereof. The tendency of the measured values of vitamin and mineral at individual stages is shown in Table 2.

## Table 2

Tendency of vitamin and mineral levels as assessed in five ranks

| Indicator substance | | Water-soluble vitamin |
|---|---|---|
| stages | Region where the indicator substance is present | Tendency of numerical figures |
| Stage 1 | Standard range or safety range | Standard or Slightly low |
| Stage 2 | Abnormal range or safety range | Slightly low |
| Stage 3 | Abnormal range | More or less low |
| Stage 4 | Abnormal range | Low |
| Stage 5 | Abnormal range | Very low |

| Indicator substance | | Fat-soluble vitamin |
|---|---|---|
| stages | Region where the indicator substance is present | Tendency of numerical figures |
| Stage 1 | Standard range | Standard or Slightly low |
| Stage 2 | Abnormal range | Slightly low or slightly high |
| Stage 3 | Abnormal range | More or less low |
| Stage 4 | Abnormal range | Low or high |
| Stage 5 | Abnormal range | Very low or very high |

| Indicator substance | | Mineral |
|---|---|---|
| stages | Region where the indicator substance is present | Tendency of numerical figures |
| Stage 1 | Standard range | Standard or Slightly low |
| Stage 2 | Abnormal range | Slightly low |
| Stage 3 | Abnormal range | More or less low, or some of minerals are high |
| Stage 4 | Abnormal range | Low |
| Stage 5 | Abnormal range | Very low, or trace amount of minerals are low |

The characteristic performance of each stage as represented in Table 2 is described here in below, together with necessary counter measures against the stage.

1. Stage 1

Vitamin and mineral are sometimes low, principally because of relative shortage thereof. Vitamin and mineral are required to be supplemented sufficiently as much as possible.

2. Stage 2

The shortage of vitamin and mineral has been continued for over one year with slight damage on human body, but with no apparent functional disorders. A certain micronutrient mineral is characteristically of shortage. It is possible that fat-soluble vitamin may simultaneously be high. Vitamin and mineral should be required to be supplemented as much as possible.

3. Stage 3

One or more minerals are rapidly increased for a short period (3 months), while the other minerals and vitamin are fairly (much) decreased. Excess mineral may be introduced into the body, except from food intake. Therefore, a great amount of vitamin should be supplemented, essentially.

4. Stage 4

Mineral shortage has been continued for a long period of time, and symptoms due to such shortage occur at some extent or apparently. On the other hand, vitamin is likely to decrease. In other words, the test subject can be called as semi-diseased patient. A long-term supplement of organic mineral and vitamin is required.

5. Stage 5

Due to the shortage of vitamin and mineral, particularly micronutrient mineral, adult diseases and pathological aging are in progress. Then, the supplement of organic mineral and vitamin is required, in addition to the therapeutic treatment.

So as to diagnose the human health state of a test subject, the progress of adult diseases and the progress of aging in the test subject, as has been described insofar, the vitamin mineral profile of the test subject is ranked as one of the stages lto 5 for assessment and appropriate treatment, whereby the vitamin mineral profile can be improved appropriately and rapidly. At each therapeutic stage, vitamin and mineral contents should be measured periodically (for example, every 10 days) and recorded on the table for assessing vitamin mineral profile, so that the effect of treatment can be assessed visually. Therefore, an appropriate therapeutic means required for the test subject can be selected and practiced.

Although the present invention has been described with reference to the particular preferred embodiments, it should be understood that various changes and modifications may be made within the scope of the invention as defined in the appended claims.

**Claims**

**1.** A method for assessing the vitamin-mineral profile in a human subject in order to allow subsequent adjustment of said vitamin-mineral profile, comprising numerically representing quantitative data for indicator substances of vitamins and minerals in blood of said subject on the basis of a blood test outcome of the subject, recording the numerical figures in a table for assessing vitamin mineral-profile, the table being a plot produced by sequentially representing indicator substance items comprising water-soluble vitamins, fat-soluble vitamins and minerals in the blood on the axis of an abscissa, representing numerical values indicating the degree of excess or shortage of indicator substances of vitamins and minerals on the axis of an ordinate, defining the standard numerical values of vitamins and minerals on the axis of the ordinate, adjusting a standard value corresponding to the normal value of each indicator substance with respect to the numerical values of the standard value, arranging the standard range of each indicator substance defined between the lower limit standard value corresponding to the lower limit of the normal range of each indicator substance and the upper limit standard value corresponding to the upper limit of the normal range thereof above and below the center where the numerical value corresponding to the standard value is placed, and individually setting the safety range and abnormal range of water-soluble vitamins and abnormal ranges of fat-soluble vitamins and minerals to assess the vitamin-mineral profile of the subject and then assessing said vitamin-mineral profile of said subject in accordance with said table in order to allow subsequent adjustment of said vitamin-mineral profile, and wherein the assessment is further based on determining which of stages 1 to 5 the subject is in based on the following tables:

| Indicator substance | | Water-soluble vitamin |
|---|---|---|
| stages | Region where the indicator substance is present | Tendency of numerical figures |
| Stage 1 | Standard range or safety range | Standard or Slightly low |
| Stage 2 | Abnormal range or safety range | Slightly low |
| Stage 3 | Abnormal range | More or less low |
| Stage 4 | Abnormal range | Low |
| Stage 5 | Abnormal range | Very low |

| Indicator substance | | Fat-soluble vitamin |
|---|---|---|
| stages | Region where the indicator substance is present | Tendency of numerical figures |
| Stage 1 | Standard range | Standard or Slightly low |
| Stage 2 | Abnormal range | Slightly low or slightly high |
| Stage 3 | Abnormal range | More or less low |
| Stage 4 | Abnormal range | Low or high |
| Stage 5 | Abnormal range | Very low or very high |

| Indicator substance | | Mineral |
|---|---|---|
| stages | Region where the indicator substance is present | Tendency of numerical figures |
| Stage 1 | Standard range | Standard or Slightly low |
| Stage 2 | Abnormal range | Slightly low |
| Stage 3 | Abnormal range | More or less low, or some of minerals are high |
| Stage 4 | Abnormal range | Low |
| Stage 5 | Abnormal range | Very low, or trace amount of minerals are low |

and if the subject is in:

Stage 1, where vitamins and minerals may be low, such vitamins and minerals are to be supplemented as much as possible, based on said plot;

Stage 2, where the shortage of vitamins and minerals has been continued for over one year with slight damage to the human subject, but with no apparent functional disorder, wherein there is a shortage of one or more micronutrient minerals, such vitamins and minerals are to be supplemented as much as possible based on said plot;

Stage 3, where one or more minerals have rapidly increased for the last three months, while other minerals and vitamins have decreased, the latter minerals are to be introduced into the body apart from food intake, and a large amount of such vitamins are to be supplemented, based on said plot;

Stage 4, where mineral shortage has been continued over a long period of time and the symptoms due to such shortage are apparent, long-term supplement of organic minerals and vitamins is to be provided, based on said plot; and

Stage 5, where due to the shortage of vitamins and minerals, adult diseases and pathological ageing progress, a supplement of such vitamins and minerals is to be provided, based on said plot.

**Patentansprüche**

1. Verfahren zur Beurteilung des Vitamin-Mineralstoff Profils bei einem menschlichen Wesen, um eine anschließende Anpassung des besagten Vitamin-Mineralstoff Profils zu erlauben, wobei das Verfahren umfasst; die numerische Darstellung von quantitativen Daten über Indikatorsubstanzen von Vitaminen und von Mineralstoffen im Blut jenes Wesens auf der Basis des Ergebnisses eines Bluttestes des betroffenen Wesens, das Aufzeichnen der numerischen Zahlen in einer Tabelle zur Beurteilung des Vitamin-Mineralstoff Profils, wobei die Tabelle eine grafische Aufzeichnung ist, welche durch eine aufeinander folgende Darstellung der Punkte der Indikatorsubstanz erzeugt worden ist und welche auf der Achse der Abszisse wasserlösliche Vitamine, fettlösliche Vitamine und Mineralstoffe in dem Blut anzeigt und auf der Achse der Ordinate numerische Werte darstellt, welche das Maß des Überschusses oder des Mangels an Indikatorsubstanzen der Vitamine und Mineralstoffen anzeigen, das Bestimmen der numerischen Standardwerte der Vitamine und Mineralstoffe auf der Achse der Ordinate, das Anpassen eines Standardwertes, welcher dem normalen Wert einer jeden Indikatorsubstanz unter Berücksichtigung der numerischen Werte des Standardwertes entspricht, das Anordnen des Standardbereiches einer jeden Indikatorsubstanz, welcher definiert wird zwischen dem unteren Standardgrenzwert entsprechend der unteren Grenze des normalen Bereiches einer jeden Indikatorsubstanz und dem oberen Standardgrenzwert entsprechend der oberen Grenze des normalen Bereiches derselben über und unter dem Zentralwert, wo der dem Standardwert entsprechende numerische Wert angeordnet ist, und das individuelle Einstellen des Sicherheitsbereiches und des abnormalen Bereiches von wasserlöslichen Vitaminen und der abnormalen Bereiche von fettlöslichen Vitaminen und Mineralstoffen, um das Vitamin-Mineralstoff Profil des Wesens zu beurteilen, und um dann das Vitamin-Mineralstoff Profil des besagten Wesens in Übereinstimmung mit der besagten Tabelle zu beurteilen, um eine anschließende Anpassung jenes Vitamin-Mineralstoff Profils zu ermöglichen, und wobei die Beurteilung weiterhin auf der Bestimmung beruht in welcher der Phasen 1 bis 5 sich das Wesen unter Zugrundelegung der folgenden Tabellen befindet:

| Indikatorsubstanz | | Wasserlösliches Vitamin |
|---|---|---|
| Phasen | Bereich, in dem die Indikatorsubstanz vorhanden ist | Tendenz der numerischen Zahlen |
| Phase 1 | Standardbereich oder Sicherheitsbereich | Standard oder schwach niedrig |
| Phase 2 | Abnormaler Bereich oder Sicherheitsbereich | Schwach niedrig |
| Phase 3 | Abnormaler Bereich | Mehr oder weniger niedrig |
| Phase 4 | Abnormaler Bereich | Niedrig |
| Phase 5 | Abnormaler Bereich | Sehr niedrig |

| Indikatorsubstanz | | Fettlösliches Vitamin |
|---|---|---|
| Phasen | Bereich, in dem die Indikatorsubstanz vorhanden ist | Tendenz der numerischen Zahlen |
| Phase 1 | Standardbereich | Standard oder schwach niedrig |
| Phase 2 | Abnormaler Bereich | Schwach niedrig oder schwach hoch |
| Phase 3 | Abnormaler Bereich | Mehr oder weniger niedrig |
| Phase 4 | Abnormaler Bereich | Niedrig oder hoch |
| Phase 5 | Abnormaler Bereich | Sehr niedrig oder sehr hoch |

| Indikatorsubstanz | | Mineralstoff |
|---|---|---|
| Phasen | Bereich, in dem die Indikatorsubstanz vorhanden ist | Tendenz der numerischen Zahlen |
| Phase 1 | Standardbereich | Standard oder schwach niedrig |
| Phase 2 | Abnormaler Bereich | Schwach niedrig |
| Phase 3 | Abnormaler Bereich | Mehr oder weniger niedrig, oder einige der Mineralstoffe sind hoch |
| Phase 4 | Abnormaler Bereich | Niedrig |
| Phase 5 | Abnormaler Bereich | Sehr niedrig, oder die Spurenmenge der Mineralstoffe ist niedrig |

und wenn sich das Wesen befindet in:

Phase 1, während welcher Vitamine und Mineralstoffe niedrig sein können, dann müssen solche Vitamine und Mineralstoffe so weit wie möglich auf der Basis jener grafischen Darstellung ergänzt werden;

Phase 2, während welcher der Mangel an Vitaminen und Mineralstoffen sich über eine Zeitdauer von einem Jahr fortgesetzt hat mit einer leichten Schädigung des menschlichen Wesens, aber mit keiner offensichtlichen, funktionalen Störung, und während welcher es einen Mangel an einem oder an mehreren Mikronährstoffmineralen gegeben hat, dann müssen solche Vitamine und Mineralstoffe so viel wie möglich auf der Basis jener grafischen Darstellung ergänzt werden;

Phase 3, während welcher eines oder mehrere der Mineralstoffe über die letzten drei Monate stark angestiegen sind, wohingegen andere Mineralstoffe und Vitamine abgenommen haben, dann müssen die letztgenannten Mineralstoffe in den Körper eingeführt werden, gesondert von der Nahrungsmittelaufnahme, und eine große Menge solcher Vitamine muss auf der Basis jener grafischen Darstellung ergänzt werden;

Phase 4, während welcher der Mangel an Mineralstoffen sich über eine lange Zeitperiode fortgesetzt hat und die auf einen solchen Mangel zurückzuführenden Symptome offensichtlich geworden sind, dann muss für eine lang andauernde Ergänzung an organischen Mineralstoffen und Vitaminen auf der Basis jener grafischen Darstellung gesorgt werden; und

Phase 5, während welcher auf Grund des Mangels an Vitaminen und Mineralstoffen Erwachsenenkrankheiten und ein pathologisches Altern zugenommen haben, dann muss eine Ergänzung an solchen Vitaminen und Mineralstoffen auf der Basis jener grafischen Darstellung bereitgestellt werden.

## Revendications

1. Procédé pour évaluer le profil vitamines-minéraux chez un sujet humain dans le but de permettre un ajustement ultérieur dudit profil vitamines-minéraux, comprenant la représentation numérique de données quantitatives pour des substances indicatrices de vitamines et de minéraux dans le sang dudit sujet sur la base de résultats d'une analyse de sang du sujet, l'enregistrement des valeurs numériques dans un tableau pour évaluer le profil vitamines-minéraux, le tableau étant un graphique produit par la représentation séquentielle d'éléments de substances indicatrices comprenant des vitamines solubles dans l'eau, des vitamines solubles dans la graisse et des minéraux dans le sang sur l'axe des abscisses, la représentation des valeurs numériques indiquant le degré d'excès ou de manque de substances indicatrices de vitamines et de minéraux sur l'axe des ordonnées, la définition des valeurs numériques de référence de vitamines et de minéraux sur l'axe des ordonnées, l'ajustement d'une valeur de référence correspondant à la valeur normale de chaque substance indicatrice par rapport aux valeurs numériques de la valeur de référence, l'arrangement de l'intervalle de référence de chaque substance indicatrice défini entre la valeur de référence de limite inférieure correspondant à la limite inférieure de l'intervalle normal de chaque substance indicatrice et la valeur de référence de limite supérieure correspondant à la limite supérieure de l'intervalle normal de celle-ci au-dessus et en dessous du centre où la valeur numérique correspondant à la valeur de référence est placée, et la fixation individuelle de l'intervalle de sécurité et de l'intervalle anormal de vitamines solubles dans l'eau et d'intervalles anormaux de vitamines solubles dans la graisse et de minéraux pour évaluer le profil vitamines-minéraux du sujet et ensuite l'évaluation dudit profil vitamines-minéraux dudit sujet conformément audit tableau dans le but de permettre un ajustement ultérieur dudit profil vitamines-minéraux, et dans lequel l'évaluation est en outre basée sur la détermination de la phase parmi les phases 1 à 5 où se trouve le sujet sur la base des tableaux suivants:

| Substance indicatrice | | Vitamine soluble dans l'eau |
|---|---|---|
| phases | Région où la substance indicatrice est présente | Tendance des valeurs numériques |
| Phase 1 | Intervalle de référence ou intervalle de sécurité | Référence ou légèrement en dessous |
| Phase 2 | Intervalle anormal ou intervalle de sécurité | Légèrement en dessous |
| Phase 3 | Intervalle anormal | Plus ou moins en dessous |
| Phase 4 | Intervalle anormal | En dessous |
| Phase 5 | Intervalle anormal | Très en dessous |

| Substance indicatrice | | Vitamine soluble dans la graisse |
|---|---|---|
| phases | Région où la substance indicatrice est présente | Tendance des valeurs numériques |
| Phase 1 | Intervalle de référence | Référence ou légèrement en dessous |
| Phase 2 | Intervalle anormal | Légèrement en dessous ou légèrement au-dessus |
| Phase 3 | Intervalle anormal | Plus ou moins en dessous |
| Phase 4 | Intervalle anormal | En dessous ou au-dessus |
| Phase 5 | Intervalle anormal | Très en dessous ou très au-dessus |

| Substance indicatrice | | Minéral |
|---|---|---|
| phases | Région où la substance indicatrice est présente | Tendance des valeurs numériques |
| Phase 1 | Intervalle de référence | Référence ou légèrement en dessous |
| Phase 2 | Intervalle anormal | Légèrement en dessous |
| Phase 3 | Intervalle anormal | Plus ou moins en dessous ou certains des minéraux sont au-dessus |
| Phase 4 | Intervalle anormal | En dessous |
| Phase 5 | Intervalle anormal | Très en dessous ou des quantités en traces de minéraux sont en dessous |

et si le sujet est dans:

la Phase 1, où les vitamines et les minéraux peuvent être en dessous, ces vitamines et ces minéraux doivent être complétés autant que possible, sur la base dudit graphique;

la Phase 2, où le manque de vitamines et de minéraux s'est poursuivi pendant plus d'un an avec un léger dommage sur le sujet humain, mais sans trouble fonctionnel apparent, où il y a un manque d'un ou de plusieurs minéraux micronutriments, ces vitamines et ces minéraux doivent être complétés autant que possible, sur la base dudit graphique;

la Phase 3, où un ou plusieurs minéraux ont rapidement augmenté pendant les trois derniers mois, tandis que d'autres minéraux et les vitamines ont diminué, ces derniers minéraux doivent être introduits dans le corps en dehors de la prise d'aliments et une grande quantité de ces vitamines doit être complétée, sur la base dudit graphique;

la Phase 4, où le manque de minéraux s'est poursuivi sur une période de temps longue et les symptômes dus à ce manque sont apparents, un complément à long terme de minéraux organiques et de vitamines doit être fourni, sur la base dudit graphique; et

la Phase 5, où, à cause du manque de vitamines et de minéraux, des maladies adultes et un vieillissement pathologique progressent, un complément de ces vitamines et ces minéraux doit être fourni, sur la base dudit graphique.

# F I G. 1

## Table for assessing Vitamin Mineral Profile

Degree of excess
or deficiency

Fat-soluble Vitamin

| | | |
|---|---|---|
| Water-soluble Vitamin | | Minerals(including micronutrient minerals) |

200
very high
175
fairly high
150

high 125
100 + α
standard 100
100 − α
low 75
fairly low
50
very low
25
0

15.0    9.2    13.8    5.0    210    50    159    30
19    2.5    4.9    4.1    82    10

1 2 3 4 5 6 7 8 9 10 11 12  13 14 15 16 17 18 19  20 21 22 23 24  25 26 27 28 29  30 31 32
indicator items

(Note) Numerical figures are represented in the unit in Table 1.

# FIG.2

Table for assessing Vitamin Mineral Profile